# EUROPEAN PATENT SPECIFICATION

(11) **EP 2 192 949 B1**
(45) Date of publication and mention of the grant of the patent: **05.09.2012**
(21) Application number: 08826093.0
(22) Date of filing: 07.07.2008
(51) Int. Cl.: A61N 1/36, A61N 1/05

(54) **RETURN ELECTRODE FOR A FLEXIBLE CIRCUIT ELECTRODE ARRAY**
NEUTRALELEKTRODE FÜR ELEKTRODENANORDNUNG EINER FLEXIBLEN SCHALTUNG
ELECTRODE DE RETOUR POUR UN RÉ SEAU D'ÉLECTRODES DE CIRCUIT FLEXIBLE

(30) Priority: 05.07.2007 US 948166 P; 24.08.2007 US 968014 P; 18.09.2007 US 973230 P; 05.10.2007 US 977929 P
(43) Date of publication of application: 09.06.2010
(73) Proprietor: Second Sight Medical Products, Inc., Sylmar, CA 91342 (US)
(72) Inventor: GREENBERG, Robert, J., Los Angeles, CA 90068 (US); NEYSMITH, Jordan, Matthew, Pasadena, CA 91101 (US); TALBOT, Neil, Hamilton, La Crescenta, CA 91214 (US); LITTLE, James, Singleton, Saugus, CA 91350 (US); MCCLURE, Kelly, H., Simi Valley, CA 93063 (US); MECH, Brian, V., Valencia, CA 91355 (US); DAI, Rongqing, Valencia, CA 91354 (US); ZHOU, David, Daomin, Saugus, CA 91390 (US); NOLAN, Gaillard, R., Oxford, MD 21643 (US)
(74) Representative: Carter, Stephen John
(86) International application number: PCT/US2008/069348
(87) International publication number: WO 2009/006636

(56) References cited:
- EP-A- 1 762 269
- WO-A-2006/119468
- US-A1- 2004 054 407
- US-A1- 2005 004 625
- US-A1- 2006 142 857
- TALUKDER, M.I.; SIY, P.; AUNER, G.W.: "Design of Return/Ground Electrode for Neural Stimulator" BIOENGINEERING CONFERENCE, 2006. PROCEEDINGS OF THE IEEE 32ND ANNUAL NORTHEAST, 15 May 2006 (2006-05-15), XP002508511

## Description

### Field of the Invention

The present invention is generally directed to neural stimulation and more specifically to an improved electrode array for neural stimulation. The present invention is more specifically directed to an improved electrode array and return electrode for visual neural stimulation.

### Background of the Invention

In 1755 LeRoy passed the discharge of a Leyden jar through the orbit of a man who was blind from cataract and the patient saw "flames passing rapidly downwards." Ever since, there has been a fascination with electrically elicited visual perception. The general concept of electrical stimulation of retinal cells to produce these flashes of light or phosphenes has been known for quite some time. Based on these general principles, some early attempts at devising prostheses for aiding the visually impaired have included attaching electrodes to the head or eyelids of patients. While some of these early attempts met with some limited success, these early prosthetic devices were large, bulky and could not produce adequate simulated vision to truly aid the visually impaired.

In the early 1930's, Foerster investigated the effect of electrically stimulating the exposed occipital pole of one cerebral hemisphere. He found that, when a point at the extreme occipital pole was stimulated, the patient perceived a small spot of light directly in front and motionless (a phosphene). Subsequently, Brindley and Lewin (1968) thoroughly studied electrical stimulation of the human occipital (visual) cortex. By varying the stimulation parameters, these investigators described in detail the location of the phosphenes produced relative to the specific region of the occipital cortex stimulated. These experiments demonstrated: (1) the consistent shape and position of phosphenes; (2) that increased stimulation pulse duration made phosphenes brighter; and (3) that there was no detectable interaction between neighboring electrodes which were as close as 2.4 mm apart.

As intraocular surgical techniques have advanced, it has become possible to apply stimulation on small groups and even on individual retinal cells to generate focused phosphenes through devices implanted within the eye itself. This has sparked renewed interest in developing methods and apparatus to aid the visually impaired. Specifically, great effort has been expended in the area of intraocular retinal prosthesis devices in an effort to restore vision in cases where blindness is caused by photoreceptor degenerative retinal diseases; such as retinitis pigmentosa and age related macular degeneration which affect millions of people worldwide.

Neural tissue can be artificially stimulated and activated by prosthetic devices that pass pulses of electrical current through electrodes on such a device. The passage of current causes changes in electrical potentials across visual neuronal membranes, which can initiate visual neuron action potentials, which are the means of information transfer in the nervous system.

Based on this mechanism, it is possible to input information into the nervous system by coding the sensory information as a sequence of electrical pulses which are relayed to the nervous system via the prosthetic device. In this way, it is possible to provide artificial sensations including vision.

One typical application of neural tissue stimulation is in the rehabilitation of the blind. Some forms of blindness involve selective loss of the light sensitive transducers of the retina. Other retinal neurons remain viable, however, and may be activated in the manner described above by placement of a prosthetic electrode device on the inner (toward the vitreous) retinal surface (epiretinal). This placement must be mechanically stable, minimize the distance between the device electrodes and the visual neurons, control the electronic field distribution and avoid undue compression of the visual neurons.

In 1986, Bullara (US Pat. No. 4,573,481) patented an electrode assembly for surgical implantation on a nerve. The matrix was silicone with embedded iridium electrodes. The assembly fit around a nerve to stimulate it.

Dawson and Radtke stimulated cat's retina by direct electrical stimulation of the retinal ganglion cell layer. These experimenters placed nine and then fourteen electrodes upon the inner retinal layer (i.e., primarily the ganglion cell layer) of two cats. Their experiments suggested that electrical stimulation of the retina with 30 to 100 µA current resulted in visual cortical responses. These experiments were carried out with needle-shaped electrodes that penetrated the surface of the retina (see also US Pat. No. 4,628,933 to Michelson).

The Michelson '933 apparatus includes an array of photosensitive devices on its surface that are connected to a plurality of electrodes positioned on the opposite surface of the device to stimulate the retina. These electrodes are disposed to form an array similar to a "bed of nails" having conductors which impinge directly on the retina to stimulate the retinal cells. US Patent 4,837,049 to Byers describes spike electrodes for neural stimulation. Each spike electrode pierces neural tissue for better electrical contact. US Patent 5,215,088 to Norman describes an array of spike electrodes for cortical stimulation. Each spike pierces cortical tissue for better electrical contact.

The art of implanting an intraocular prosthetic device to electrically stimulate the retina was advanced with the introduction of retinal tacks in retinal surgery. De Juan, et al. at Duke University Eye Center inserted retinal tacks into retinas in an effort to reattach retinas that had detached from the underlying choroid, which is the source of blood supply for the outer retina and thus the photoreceptors. See, e.g., E. de Juan, et al., 99 Am. J. Ophthalmol. 272 (1985). These retinal tacks have proved to be biocompatible and remain embedded in the retina, and choroid/sclera, effectively pinning the retina against the choroid and the posterior aspects of the globe. Retinal tacks are one way to attach a retinal electrode array to the retina. US Patent 5,109,844 to de Juan describes a flat electrode array placed against the retina for visual stimulation. US Patent 5,935,155 to Humayun describes a retinal prosthesis for use with the flat retinal array described in de Juan.

US 2006/0142857 discloses an artificial retinal device, EP 1762269 discloses a visual prosthesis, and WO 2006/119468 discloses a retinal prosthesis.

Implanted stimulation devices usually stimulate the nerve or muscle tissue with biphasic electrical current pulses in either mono-polar or bipolar electrode configurations. In a bipolar configuration, the stimulating currents flow between two active electrodes or electrode groups which may be dynamically selected. In a mono-polar configuration, the stimulation currents flow in one phase from the active electrodes through the tissue being stimulated to the common electrode (CE), also called return electrode. The currents flow in the other phase in the reversed direction to balance the charge. In this case, the direction of the current flow is significantly affected by the relative placements or positioning of the active electrodes and the common electrode.

If an electrode array is placed on the surface of the tissue being stimulated without a ready method of confinement the array may not be in good contact with the retina tissue, or it drifts away from the retina. This applies especially for epi-retinal prosthesis where the stimulating electrode array is placed on the surface of the retina in the vitreous. If the return electrode is placed far away from the active electrode array, the current paths from the active stimulating electrodes will change because of the significant difference between the impedances of the retina tissue and of the vitreous and body fluids. As a result, there may not be enough current density passing through the neuronal cells in the retina tissue to cause a response, or the response may change when the array is moved.

In order to solve this problem, a large return electrode can be placed outside of the eyeball and directly under the active array. Thus the stimulating currents shall pass from the active electrodes through the tissue being stimulated. However, if the active electrodes are lifted from the tissue surface, the responses elicited by the stimulation of the individual electrodes may not be differentiable or distinctive because of the diffusive current paths from the lifted electrodes, resulting great reduction of effective resolution for percepts. Another possible method is to place the active electrode array underneath the retina while placing the return electrode in the vitreous. This sub-retina approach presents significant surgical difficultness when the device and array are implanted.

### Summary of the Invention

In a visual prosthesis electrodes stimulate retinal tissue to induce the perception of light to a user implanted with the prosthesis. The prosthesis must have a return, or common, electrode to make a complete circuit with the retinal tissue. To avoid stimulating tissue with the return electrode, it is advantageous that the electrode is large and in tissue less sensitive to electrical stimulation.

The novel features of the invention are set forth with particularity in the appended claims. The invention is defined by claim 1 and will be best understood from the following description when read in conjunction with the accompanying drawings.

This invention involves the use of a multi-electrode array as multi-return electrodes placed outside the eyeball and directly under the active epi-retina array. It will greatly reduce the sensitivity of the electrode-tissue distance to stimulation results. The requirement of firm contact of electrodes to the retina is relieves. Therefore the risk of damaging the delicate retina by placing the electrode array on its surface is reduced. The return electrode can be implanted routinely during the surgery.

Performance of electrical stimulation of neurons may depend on the electric field distribution from each electrode. It is believed that a more focused electric field with improved performance reduces threshold charge. In this sense a focal and return electrode or electrode array may be highly advantageous.

For retinal neuron stimulators of the epiretinal configuration, a focal return electrode or array would be placed posterior of the sclera aligned with the epiretinal stimulation array. Alignment could be achieved using concentric fixation structures, such as a tack, or the return electrode could be contained within a somewhat rigid housing connected to the other extra ocular implant components, like band or coil.

This invention involves an electrode array used as multi-return electrodes. The method involves controlling the stimulating current flow between the active electrodes and the return electrodes, and alternative ways to implement the multi-return electrode array. The muti-return electrode comprises an array of electrode discs with larger sizes than the discs of the active electrodes. The electrode discs can be made of safe electrode materials similar to or same as that of the active electrodes. The number of return electrode discs in the array can be a fraction of the active electrodes and may vary depending on the manufacturing flexibility.

### Brief Description of the Drawings

FIG. 1 is a perspective view of the implanted portion of the preferred retinal prosthesis.
FIG. 2 is a side view of the implanted portion of the preferred retinal prosthesis showing the fan tail in more detail.
FIGS. 3A - 3E depict molds for forming the flexible circuit array in a curve.
FIG. 4 depicts an alternate view of the invention with ribs to help maintain curvature and prevent retinal damage.
FIG. 5 depicts an alternate view of the invention with ribs to help maintain curvature and prevent retinal damage fold of the flexible circuit cable and a fold A between the circuit electrode array and the flexible circuit cable.
FIG. 6 depicts a cross-sectional view of the prosthesis shown insight of the eye with an angle in the fold of the flexible circuit cable and a fold between the circuit electrode array and the flexible circuit cable.
FIG. 7 depicts the implanted portion including a twist in the array to reduce the width of a sclerotomy and a sleeve to promote sealing of the sclerotomy.
FIG. 8 depicts the flexible circuit array before it is folded and attached to the implanted portion.
FIG. 9 depicts the flexible circuit array folded.
FIG. 10 depicts a flexible circuit array with a protective skirt.
FIG. 11 depicts a flexible circuit array with a protective skirt bonded to the back side of the flexible circuit array.
FIG. 12 depicts a flexible circuit array with a protective skirt bonded to the front side of the flexible circuit array.
FIG. 13 depicts a flexible circuit array with a protective skirt bonded to the back side of the flexible circuit array and molded around the edges of the flexible circuit array.
FIG. 14 depicts a flexible circuit array with a protective skirt bonded to the back side of the flexible circuit array and molded around the edges of the flexible circuit array and flush with the front side of the array.
FIG. 15 is an enlarged view of a single electrode within the flexible circuit electrode array.
FIG. 16 depicts the flexible circuit array before it is folded and attached to the implanted portion containing an additional fold between the flexible electrode array and the flexible cable.
FIG. 17 depicts the flexible circuit array of FIG. 16 folded containing an additional fold between the flexible electrode array and the flexible cable.
FIG. 18 depicts a flexible circuit array of FIG. 17 with a protective skirt and containing an additional fold between the flexible electrode array and the flexible cable.
FIG. 19 depicts a top view of a flexible circuit array and flexible circuit cable showing an additional horizontal angel between the flexible electrode array and the flexible cable.
FIG. 20 depicts another variation without the horizontal angel between the flexible electrode array and the flexible cable but with an orientation of the electrodes in the flexible electrode array as shown for the variation in FIG. 19.
FIG. 21 depicts a top view of a flexible circuit array and flexible circuit cable wherein the array contains a slit along the length axis.
FIG. 22 depicts a top view of a flexible circuit array and flexible circuit cable wherein the array contains a slit along the length axis with a two attachment points.
FIG. 23 depicts a flexible circuit array with a protective skirt bonded to the back side of the flexible circuit array with a progressively decreasing radius.
FIG. 24 depicts a flexible circuit array with a protective skirt bonded to the front side of the flexible circuit array with a progressively decreasing radius.
FIG. 25 depicts a flexible circuit array with a protective skirt bonded to the back side of the flexible circuit array and molded around the edges of the flexible circuit array with a progressively decreasing radius.
FIG. 26 depicts a flexible circuit array with a protective skirt bonded to the back side of the flexible circuit array and molded around the edges of the flexible circuit array and flush with the front side of the array with a progressively decreasing radius.
FIG. 27 depicts a side view of the flexible circuit array with a skirt containing a grooved and rippled pad instead a suture tab.
FIG. 28 depicts a side view of the enlarged portion of the skirt shown in FIG. 27 containing a grooved and rippled pad and a mattress suture.
FIG. 29 depicts a flexible circuit array with a protective skirt bonded to the front side of the flexible circuit array with individual electrode windows.
FIG. 30 depicts a flexible circuit array with a protective skirt bonded to the back side of the flexible circuit array and molded around the edges of the flexible circuit array with individual electrode windows.
FIGS. 31-36 show several surfaces to be applied on top of the cable.
FIG. 37 depicts the top view of the flexible circuit array being enveloped within an insulating material.
FIG. 38 depicts a cross-sectional view of the flexible circuit array being enveloped within an insulating material.
FIG. 39 depicts a cross-sectional view of the flexible circuit array being enveloped within an insulating material with open electrodes and the material between the electrodes.
FIG. 40 depicts a cross-sectional view of the flexible circuit array being enveloped within an insulating material with open electrodes.
FIG. 41 depicts a cross-sectional view of the flexible circuit array being enveloped within an insulating material with electrodes on the surface of the material.
FIG. 42 depicts a cross-sectional view of the flexible circuit array being enveloped within an insulating material with electrodes on the surface of the material insight the eye with an angle in the fold of the flexible circuit cable and a fold between the circuit electrode array and the flexible circuit cable.
FIG. 43 depicts a side view of the enlarged portion of the flexible circuit array being enveloped within an insulating material with electrodes on the surface of the material insight the eye.
FIG. 44 shows of front view of a cochlear electrode array according to the present invention.
FIG. 45 shows a side view of a cochlear electrode array according to the present invention.
FIG. 46 shows a cochlear electrode array according to the present invention as implanted in the cochlea.
FIG. 47 is the preferred electrode array with the return on the front of the cable outside the eye.
FIG. 48 is the preferred electrode array with the return on front of the cable inside the eye.
FIG. 49 is the preferred electrode array with the return on the back of the cable outside the eye.
FIG. 50 is the preferred electrode array with the return on the back of the cable inside the eye.
FIG. 51 is the preferred electrode array with the return on the back of the electrode array.
FIG. 52 is the preferred electrode array with the return on a separate cable inside the eye.
FIG. 53 is the preferred electrode array with the return on the face of the electrode array in a horseshoe pattern around the stimulating electrodes.
FIG. 54 is the preferred electrode array with the return around the tack hole and in electrical contact with the tack.
FIG. 55 is the preferred visual prosthesis with the return electrode on the back of the secondary coil against the sclera.
FIG. 56 depicts a cross sectional vie of epi-retina prosthesis configuration using multi-return electrode array placed on the eye wall outside the eye.
FIG. 57 depicts an enlarged portion of the configuration shown in Figure 56.
FIG. 58 depicts a cross sectional view of an eye with stimulating and return rod electrodes.
FIG. 59 depicts a cross sectional view of an eye with stimulating and return pointed electrodes.
FIG. 60 depicts an elevated cross-sectional view of insulated return electrodes.
FIG. 61 depicts a top view on a focal return electrode having a similar perimeter as the electrode array.
FIG. 62 depicts a top view on a focal return electrode matching individual electrodes.
FIG. 63 depicts a top view on a focal return electrode matching small groups of electrodes.
FIG. 64 depicts a cross sectional view of a packaging for an implantable device, which consists of a discrete package.
FIG. 65 depicts a cross sectional view of a packaging for an implantable device, which consists of a flexible circuit like structure.

### Detailed Description of the Preferred Embodiments

The following description is of the best mode presently contemplated for carrying out the invention. This description is not to be taken in a limiting sense, but is made merely for the purpose of describing the general principles of the invention. The scope of the invention should be determined with reference to the claims.

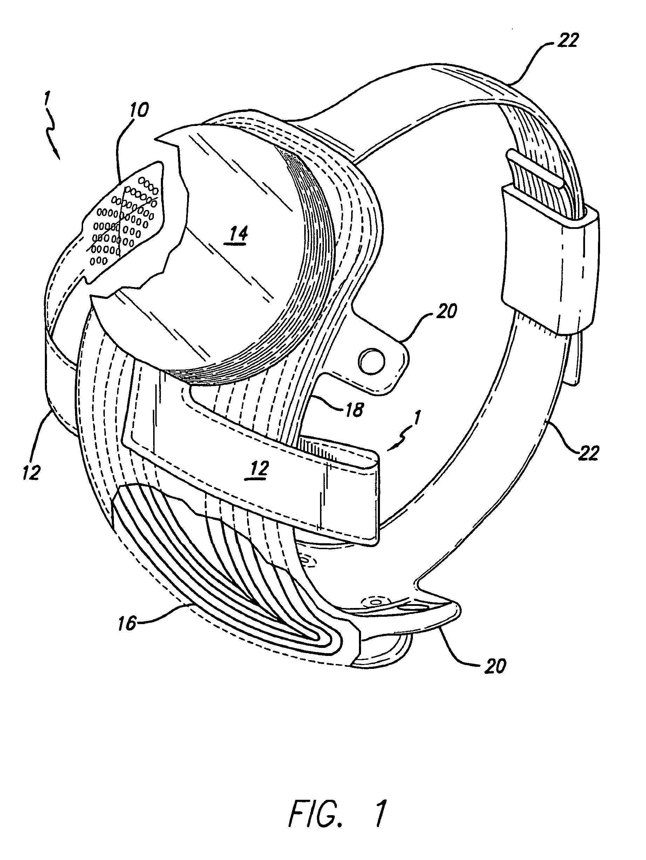

Fig. 1 shows a perspective view of the implanted portion of the preferred retinal prosthesis. A flexible circuit 1 includes a flexible circuit electrode array 10 which is mounted by a retinal tack (not shown) or similar means to the epiretinal surface. The flexible circuit electrode array 10 is electrically coupled by a flexible circuit cable 12, which pierces the sclera and is electrically coupled to an electronics package 14, external to the sclera.

The electronics package 14 is electrically coupled to a secondary inductive coil 16. Preferably the secondary inductive coil 16 is made from wound wire. Alternatively, the secondary inductive coil 16 may be made from a flexible circuit polymer sandwich with wire traces deposited between layers of flexible circuit polymer. The electronics package 14 and secondary inductive coil 16 are held together by a molded body 18. The molded body 18 may also include suture tabs 20. The molded body 18 narrows to form a strap 22 which surrounds the sclera and holds the molded body 18, secondary inductive coil 16, and electronics package 14 in place. The molded body 18, suture tabs 20 and strap 22 are preferably an integrated unit made of silicone elastomer. Silicone elastomer can be formed in a pre-curved shape to match the curvature of a typical sclera. However, silicone remains flexible enough to accommodate implantation and to adapt to variations in the curvature of an individual sclera. The secondary inductive coil 16 and molded body 18 are preferably oval shaped. A strap 22 can better support an oval shaped coil.

It should be noted that the entire implant is attached to and supported by the sclera. An eye moves constantly. The eye moves to scan a scene and also has a jitter motion to improve acuity. Even though such motion is useless in the blind, it often continues long after a person has lost their sight. By placing the device under the rectus muscles with the electronics package in an area of fatty tissue between the rectus muscles, eye motion does not cause any flexing which might fatigue, and eventually damage, the device.

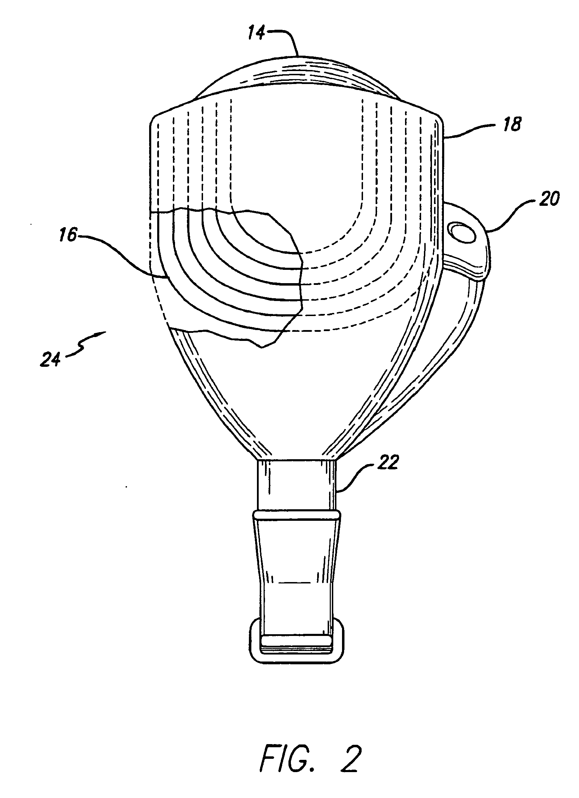

Fig. 2 shows a side view of the implanted portion of the retinal prosthesis, in particular, emphasizing the fan tail 24. When implanting the retinal prosthesis, it is necessary to pass the strap 22 under the eye muscles to surround the sclera. The secondary inductive coil 16 and molded body 18 must also follow the strap 22 under the lateral rectus muscle on the side of the sclera. The implanted portion of the retinal prosthesis is very delicate. It is easy to tear the molded body 18 or break wires in the secondary inductive coil 16. In order to allow the molded body 18 to slide smoothly under the lateral rectus muscle, the molded body 18 is shaped in the form of a fan tail 24 on the end opposite the electronics package 14.

The flexible circuit 1 is a made by the following process. First, a layer of polymer (such as polyimide, fluoro-polymers, silicone or other polymers) is applied to a support substrate (not part of the array) such as glass. Layers may be applied by spinning, meniscus coating, casting, sputtering or other physical or chemical vapor deposition, or similar process. Subsequently, a metal layer is applied to the polymer. The metal is patterned by photolithographic process. Preferably, a photo-resist is applied and patterned by photolithography followed by a wet etch of the unprotected metal. Alternatively, the metal can be patterned by lift-off technique, laser ablation or direct write techniques.

It is advantageous to make this metal thicker at the electrode and bond pad to improve electrical continuity. This can be accomplished through any of the above methods or electroplating. Then, the top layer of polymer is applied over the metal. Openings in the top layer for electrical contact to the electronics package 14 and the electrodes may be accomplished by laser ablation or reactive ion etching (RIE) or photolithography and wet etch. Making the electrode openings in the top layer smaller than the electrodes promotes adhesion by avoiding delamination around the electrode edges.

The pressure applied against the retina by the flexible circuit electrode array is critical. Too little pressure causes increased electrical resistance between the array and retina. It should be noted that while the present invention is described in terms of application to the retina, the techniques described are equally applicable to many forms of neural stimulation. Application to the retina requires a convex spherical curve. Application to the cochlea requires a constant curve in one dimension and a spiral curve in the other. Application to the cerebral cortex requires a concave spherical curve. Cortical stimulation is useful for artificial vision or hearing, touch and motor control for limb prostheses, deep brain stimulation for Parkinson's disease and multiple sclerosis, and many other applications.

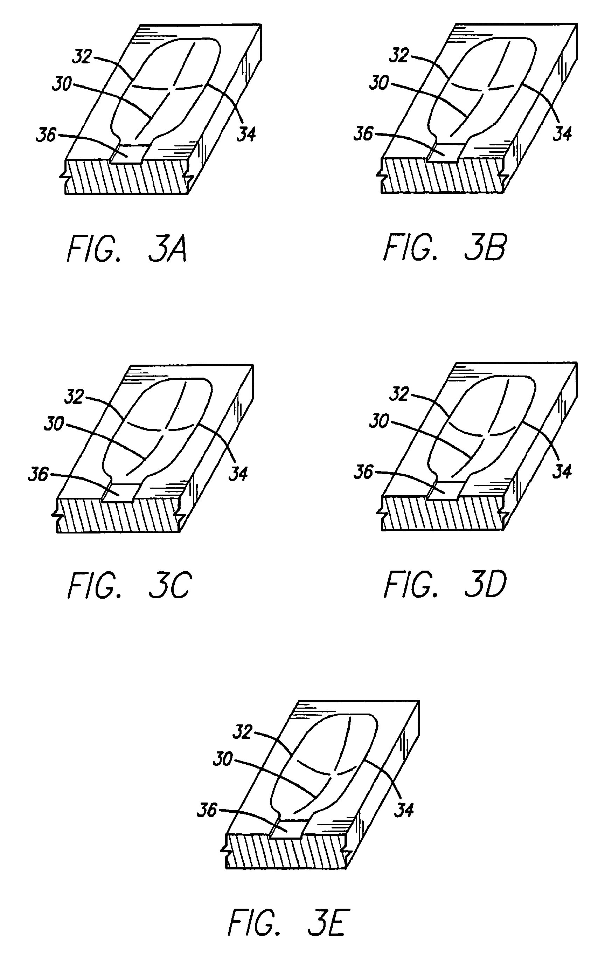

Common flexible circuit fabrication techniques such as photolithography generally require that a flexible circuit electrode array be made flat. Since the retina is spherical, a flat array will necessarily apply more pressure near its edges, than at its center. With most polymers, it is possible to curve them when heated in a mold. By applying the right amount of heat to a completed array, a curve can be induced that matches the curve of the retina. To minimize warping, it is often advantageous to repeatedly heat the flexible circuit in multiple molds, each with a decreasing radius. Fig. 3 illustrates a series of molds according to the preferred embodiment. Since the flexible circuit will maintain a constant length, the curvature must be slowly increased along that length. As the curvature 30 decreases in successive molds (Figs. 3A- 3E) the straight line length between ends 32 and 34, must decrease to keep the length along the curvature 30 constant, where mold 3E approximates the curvature of the retina or other desired neural tissue. The molds provide a further opening 36 for the flexible circuit cable 12 of the array to exit the mold without excessive curvature.

It should be noted that suitable polymers include thermoplastic materials and thermoset materials. While a thermoplastic material will provide some stretch when heated a thermoset material will not. The successive molds are, therefore, advantageous only with a thermoplastic material. A thermoset material works as well in a single mold as it will with successive smaller molds. It should be noted that, particularly with a thermoset material, excessive curvature in three dimensions will cause the polymer material to wrinkle at the edges. This can cause damage to both the array and the retina. Hence, the amount of curvature is a compromise between the desired curvature, array surface area, and the properties of the material.

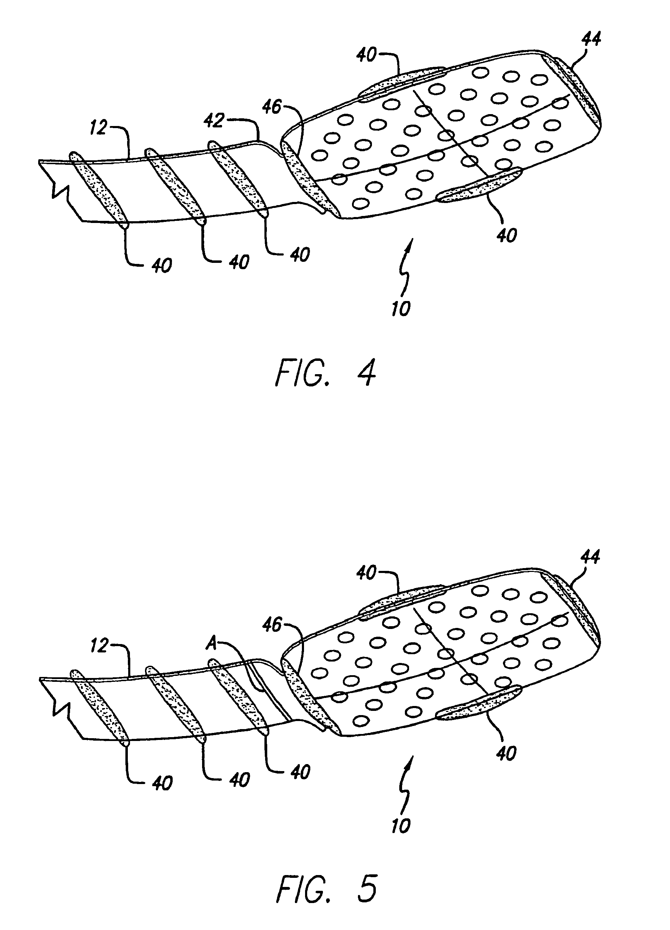

Referring to Fig. 4, the edges of the polymer layers are often sharp. There is a risk that the sharp edges of a flexible circuit will cut into delicate retinal tissue. It is advantageous to add a soft material, such as silicone, to the edges of a flexible circuit electrode array to round the edges and protect the retina. Silicone around the entire edge is preferable, but may make the flexible circuit less flexible. So, another embodiment as depicted in Fig. 4 has discrete silicone bumpers or ribs to hold the edge of the flexible circuit electrode array away from the retinal tissue. Curvature 40 fits against the retina. The leading edge 44 is most likely to cause damage and is therefore fit with molded silicone bumper. Also, edge 46, where the array lifts off the retina can cause damage and should be fit with a bumper. Any space along the side edges of curvature 40 may cause damage and may be fit with bumpers as well. It is also possible for the flexible circuit cable 12 of the electrode array to contact the retina. It is, therefore, advantageous to add periodic bumpers along the flexible circuit cable 12.

It is also advantageous to create a reverse curve or service loop in the flexible circuit cable 12 of the flexible circuit electrode array to gently lift the flexible circuit cable 12 off the retina and curve it away from the retina, before it passes through the sclera at a sclerotomy. It is not necessary to heat curve the service loop as described above, the flexible circuit electrode array can simply be bent or creased upon implantation. This service loop reduces the likelihood of any stress exerted extraocularly from being transmitted to the electrode region and retina. It also provides for accommodation of a range of eye sizes.

With existing technology, it is necessary to place the implanted control electronics outside of the sclera, while a retinal flexible circuit electrode array must pass through the sclera to in order be inside the eye and contact the retina. The sclera is cut through at the pars plana, forming a sclerotomy, and the flexible circuit passed through the sclerotomy. A flexible circuit is thin but wide. The more electrode conductors, the wider the flexible circuit must be. It may be difficult to seal a sclerotomy over a flexible circuit wide enough to support enough conductors for a high resolution array unless multiple conductor layers are employed. A narrow sclerotomy is preferable.

Fig. 5 depicts a further embodiment of the part of the prosthesis shown in Fig. 4 with a fold A between the flexible circuit electrode array 10 and the flexible circuit cable 12. The angle in the fold A also called ankle has an angle of 1°-180°, preferably 80°-120°. The fold A is advantageous since it reduces tension and enables an effective attachment of the flexible electrode circuit array 10 to the retina.

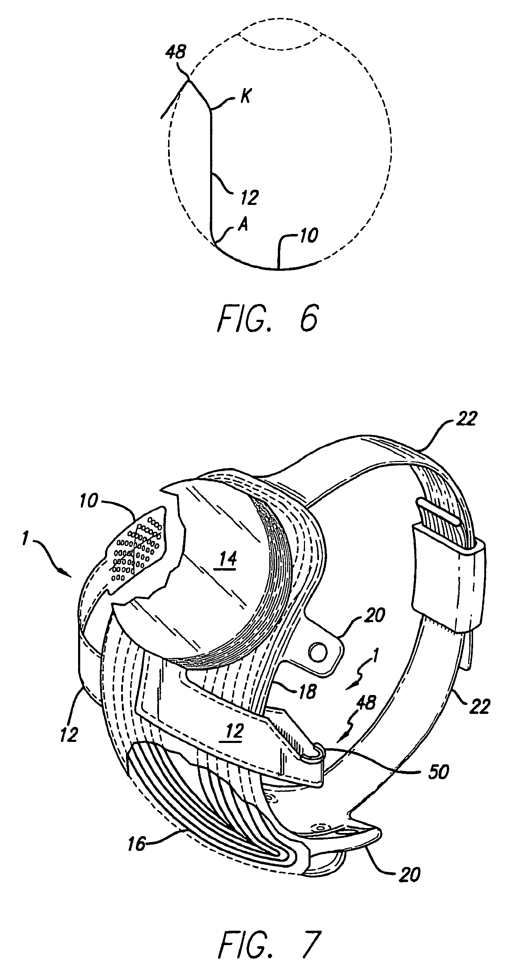

Fig. 6 depicts a side view of the prosthesis insight of the eye with an angle K of the flexible circuit cable 12 and a fold A between the circuit electrode array 10 and the flexible circuit cable 12. Fold K may alternatively be located at the sclerotomy. The angle K is about 45°-180° and preferably 80°-100°. The fold K also called knee is advantageous because it decreases force which would be applied by the flexible circuit cable 12 on the electrode region 10. Since the magnitude and direction of this force varies greatly with eye size it is best to minimize the effect of this force on the important electrode region of the flexible circuit electrode array 10 in order to maintain equal performance across a range of eye sizes. Additionally the fold K keeps the flexible circuit cable 12 from blocking the surgeon's view of the electrode region 10 during surgery. Visualization of the electrode region 10 during surgery is very important during the attachment of the flexible circuit electrode array to the retina in order to permit correct positioning.

Fig. 7 shows the implanted portion of the retinal prosthesis including the additional feature of a twist or fold 48 in the flexible circuit cable 12, where the flexible circuit cable 12 passes through the sclera (sclerotomy). The twist may be a simple sharp twist, or fold 48; or it may be a longer twist, forming a tube. While the tube is rounder, it reduces the flexibility of the flexible circuit. A simple fold 48 reduces the width of the flexible circuit with only minimal impact on flexibility.

Further, silicone or other pliable substance may be used to fill the center of the tube or fold 48 formed by the twisted flexible circuit cable 12. Further it is advantageous to provide a sleeve or coating 50 that promotes sealing of the sclerotomy. Polymers such as polyimide, which may be used to form the flexible circuit cable 12 and flexible circuit electrode array 10, are generally very smooth and do not promote a good bond between the flexible circuit cable 12 and scleral tissue. A sleeve or coating of polyester, collagen, silicone, Gore-tex or similar material would bond with scleral tissue and promote healing. In particular, a porous material will allow scleral tissue to grow into the pores promoting a good bond.

Alternatively, the flexible circuit electrode array 10 may be inserted through the sclera, behind the retina and placed between the retina and choroid to stimulate the retina subretinally. In this case, it is advantageous to provide a widened portion, or stop, of the flexible circuit cable 12 to limit how far the flexible circuit electrode array is inserted and to limit the transmission of stress through the sclera. The stop may be widening of the flexible circuit 1 or it may be added material such as a bumper or sleeve.

Human vision provides a field of view that is wider than it is high. This is partially due to fact that we have two eyes, but even a single eye provides a field of view that is approximately 90° high and 140° to 160° degrees wide. It is therefore, advantageous to provide a flexible circuit electrode array 10 that is wider than it is tall. This is equally applicable to a cortical visual array. In which case, the wider dimension is not horizontal on the visual cortex, but corresponds to horizontal in the visual scene.

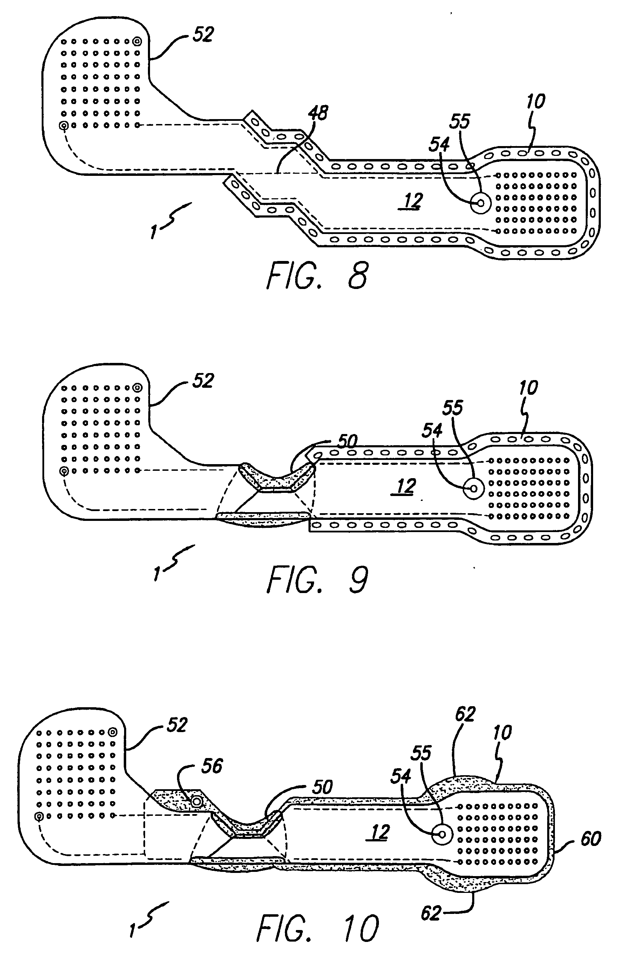

Fig. 8 shows the flexible circuit electrode array prior to folding and attaching the array to the electronics package. At one end of the flexible circuit cable 12 is an interconnection pad 52 for connection to the electronics package. At the other end of the flexible circuit cable 12 is the flexible circuit electrode array 10. Further, an attachment point 54 is provided near the flexible circuit electrode array 10. A retinal tack (not shown) is placed through the attachment point 54 to hold the flexible circuit electrode array 10 to the retina. A stress relief 55 is provided surrounding the attachment point 54. The stress relief 55 may be made of a softer polymer than the flexible circuit, or it may include cutouts or thinning of the polymer to reduce the stress transmitted from the retina tack to the flexible circuit electrode array 10. The flexible circuit cable 12 is formed in a dog leg pattern so than when it is folded at fold 48 it effectively forms a straight flexible circuit cable 12 with a narrower portion at the fold 48 for passing through the sclerotomy.

Fig. 9 shows the flexible circuit electrode array after the flexible circuit cable 12 is folded at the fold 48 to form a narrowed section. The flexible circuit cable 12 may include a twist or tube shape as well. With a retinal prosthesis as shown in Fig. 1, the bond pad 52 for connection to the electronics package 14 and the flexible circuit electrode array 10 are on opposite side of the flexible circuit. This requires patterning, in some manner, both the base polymer layer and the top polymer layer. By folding the flexible circuit cable 12 of the flexible circuit electrode array 10, the openings for the bond pad 52 and the electrodes are on the top polymer layer and only the top polymer layer needs to be patterned.

Also, since the narrowed portion of the flexible circuit cable 12 pierces the sclera, shoulders formed by opposite ends of the narrowed portion help prevent the flexible circuit cable 12 from moving through the sclera. It may be further advantageous to add ribs or bumps of silicone or similar material to the shoulders to further prevent the flexible circuit cable 12 from moving through the sclera.

Further it is advantageous to provide a suture tab 56 in the flexible circuit body near the electronics package to prevent any movement in the electronics package from being transmitted to the flexible circuit electrode array 10. Alternatively, a segment of the flexible circuit cable 12 can be reinforced to permit it to be secured directly with a suture.

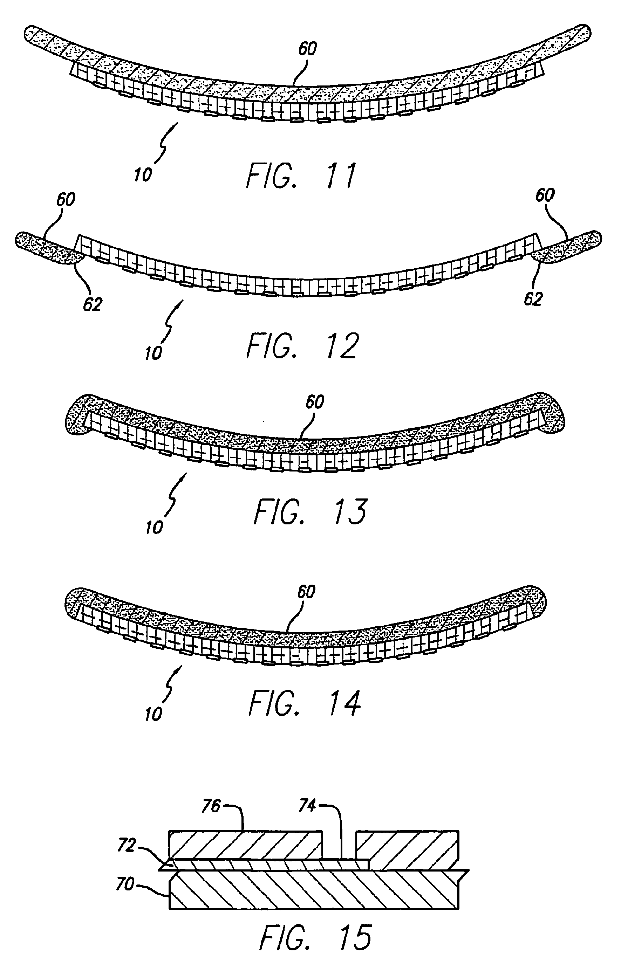

An alternative to the bumpers described in Fig. 4, is a skirt of silicone or other pliable material as shown in Figs. 11, 12, 13 and 14. A skirt 60 covers the flexible circuit electrode array 10, and extends beyond its edges. It is further advantageous to include wings 62 adjacent to the attachment point 54 to spread any stress of attachment over a larger area of the retina. There are several ways of forming and bonding the skirt 60. The skirt 60 may be directly bonded through surface activation or indirectly bonded using an adhesive.

Alternatively, a flexible circuit electrode array 10 may be layered using different polymers for each layer. Using too soft of a polymer may allow too much stretch and break the metal traces. Too hard of a polymer may cause damage to delicate neural tissue. Hence a relatively hard polymer, such a polyimide may be used for the bottom layer and a relatively softer polymer such a silicone may be used for the top layer including an integral skirt to protect delicate neural tissue. The said top layer is the layer closest to the retina.

The simplest solution is to bond the skirt 60 to the back side (away from the retina) of the flexible circuit electrode array 10 as shown in Fig. 11. While this is the simplest mechanical solution, sharp edges of the flexible circuit electrode array 10 may contact the delicate retina tissue. Bonding the skirt to the front side (toward the retina) of the flexible circuit electrode array 10, as shown in Fig. 12, will protect the retina from sharp edges of the flexible circuit electrode array 10. However, a window 62 must be cut in the skirt 60 around the electrodes. Further, it is more difficult to reliably bond the skirt 60 to the flexible circuit electrode array 10 with such a small contact area. This method also creates a space between the electrodes and the retina which will reduce efficiency and broaden the electrical field distribution of each electrode. Broadening the electric field distribution will limit the possible resolution of the flexible circuit electrode array 10.

Fig. 13 shows another structure where the skirt 60 is bonded to the back side of the flexible circuit electrode array 10, but curves around any sharp edges of the flexible circuit electrode array 10 to protect the retina. This gives a strong bond and protects the flexible circuit electrode array 10 edges. Because it is bonded to the back side and molded around the edges, rather than bonded to the front side, of the flexible circuit electrode array 10, the portion extending beyond the front side of the flexible circuit electrode array 10 can be much smaller. This limits any additional spacing between the electrodes and the retinal tissue.

Fig. 14 shows a flexible circuit electrode array 10 similar to Fig. 13, with the skirt 60, flush with the front side of the flexible circuit electrode array 10 rather than extending beyond the front side. While this is more difficult to manufacture, it does not lift the electrodes off the retinal surface as with the array in Fig. 10. It should be noted that Figs. 11, 13, and 14 show skirt 60 material along the back of the flexible circuit electrode array 10 that is not necessary other than for bonding purposes. If there is sufficient bond with the flexible circuit electrode array 10, it may advantageous to thin or remove portions of the skirt 60 material for weight reduction.

Referring to Fig. 15, the flexible circuit electrode array 10 is manufactured in layers. A base layer of polymer 70 is laid down, commonly by some form of chemical vapor deposition, spinning, meniscus coating or casting. A layer of metal 72 (preferably platinum) is applied to the polymer base layer 70 and patterned to create electrodes 74 and traces for those electrodes. Patterning is commonly done by photolithographic methods. The electrodes 74 may be built up by electroplating or similar method to increase the surface area of the electrode 74 and to allow for some reduction in the electrodes 74 over time. Similar plating may also be applied to the bond pads 52 (Figs. 8-10). A top polymer layer 76 is applied over the metal layer 72 and patterned to leave openings for the electrodes 74, or openings are created later by means such as laser ablation. It is advantageous to allow an overlap of the top polymer layer 76 over the electrodes 74 to promote better adhesion between the layers, and to avoid increased electrode reduction along their edges. The overlapping top layer promotes adhesion by forming a clamp to hold the metal electrode between the two polymer layers. Alternatively, multiple alternating layers of metal and polymer may be applied to obtain more metal traces within a given width.

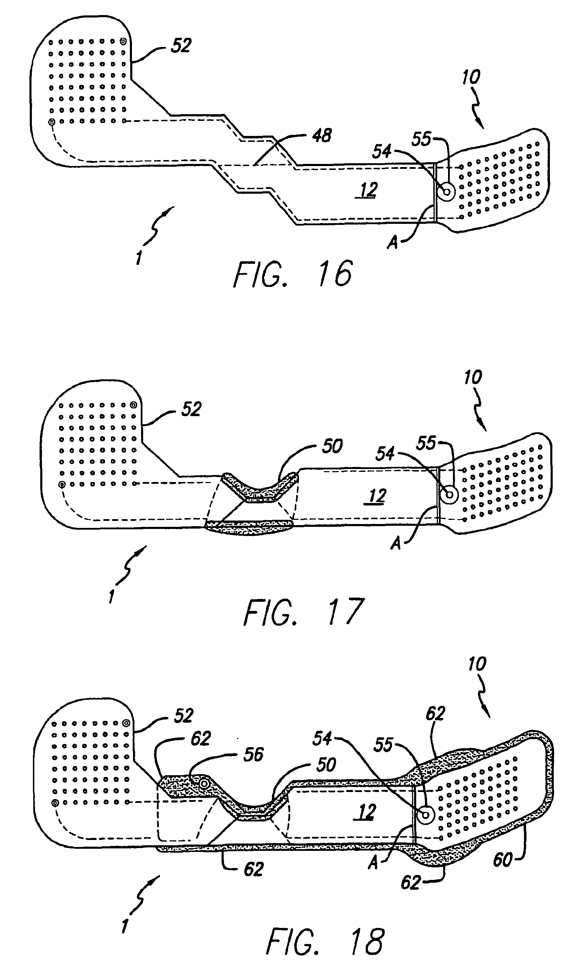

Fig. 16 depicts the flexible circuit array 12 before it is folded and attached to the implanted portion containing an additional fold A between the flexible electrode array 12 and the flexible cable 10. The angle in the fold A also called ankle has an angle of 1°-180°, preferably 80°-120°. The ankle is advantageous in the process of inserting the prostheses in the eye and attaching it to the retina.

Fig. 17 depicts the flexible circuit array 12 containing an additional fold A between the flexible electrode array 12 and the flexible cable 10. The flexible circuit array as shown in Figs. 8 and 16 differ by the fold A from each other.

Fig. 18 depicts a flexible circuit array of Fig. 17 with a protective skirt 60 and containing an additional fold A between the flexible electrode array and the flexible cable. The flexible circuit array as shown in Figs. 10 and 18 differ by the fold A from each other.

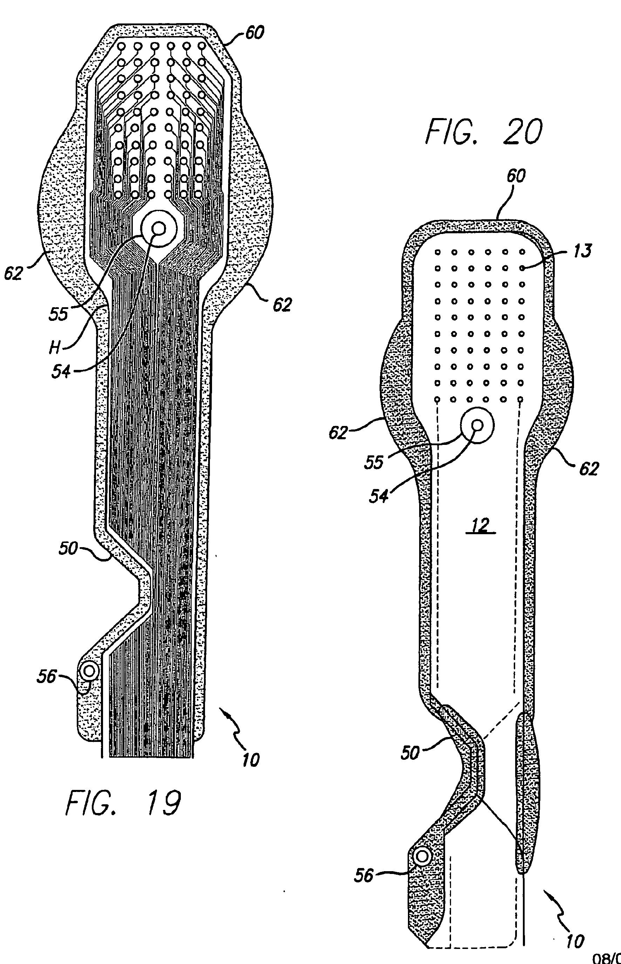

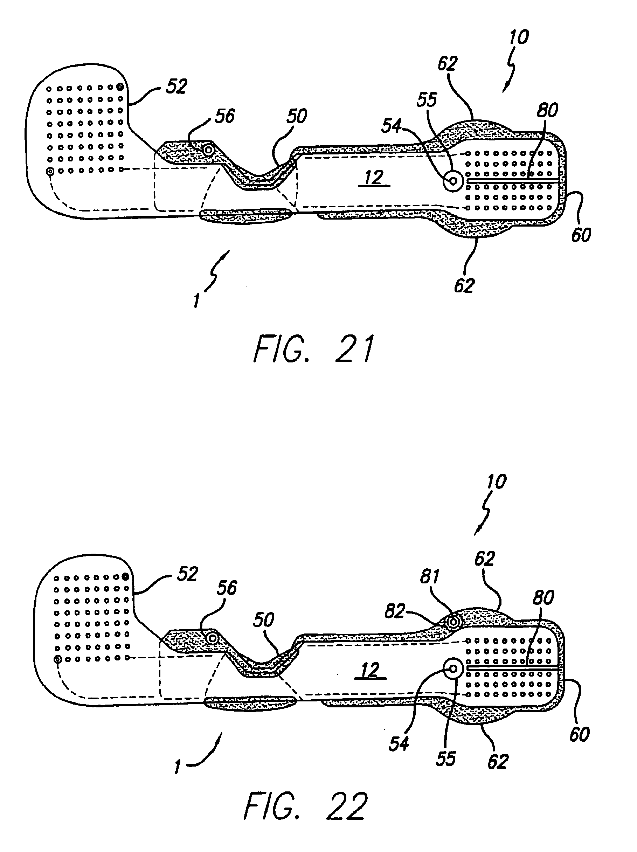

Fig. 21 depicts a top view of a flexible circuit array and flexible circuit cable as shown in Figs. 10, 18, 19, and 20 wherein the array in Fig. 21 contains a slit along the length axis.

Fig. 22 depicts a skirt of silicone or other pliable material as shown in Fig. 10 to 14. A skirt 60 covers the flexible circuit electrode array 10, and extends beyond its edges. In this embodiment of the present invention the flexible circuit electrode array contains a slit 80 along the lengths axis. Further, according to this embodiment the skirt of silicone or other pliable material contains preferably at least two attachment points 81 and stress relieves 82 are provided surrounding the attachment points 81. The attachment points 81 are located preferably on the skirt 60 outside the flexible circuit electrode 10 and are positioned apart as far as possible from each other. The secondary tack 81 is far enough away from the first tack location 54 not to cause tenting, therefore fibrosis between the two tacks which cause a traction detachment of the retina. Furthermore, the polyimide is completely between the two tacks, which also reduce the possibility of tenting. Also, this orientation of tacks keeps the tacks away from the axons, which arise from the ganglion cells which are tried to be activated. They are away from the raffe. The wings act like external tabs or strain relieves. The multiple tacks prevent rotation of the array. Alternatively the secondary tack could be placed at an attachment point at 83.

The stress relief 82 may be made of a softer polymer than the flexible circuit, or it may include cutouts or thinning of the polymer to reduce the stress transmitted from the retina tack to the flexible circuit electrode array 10.

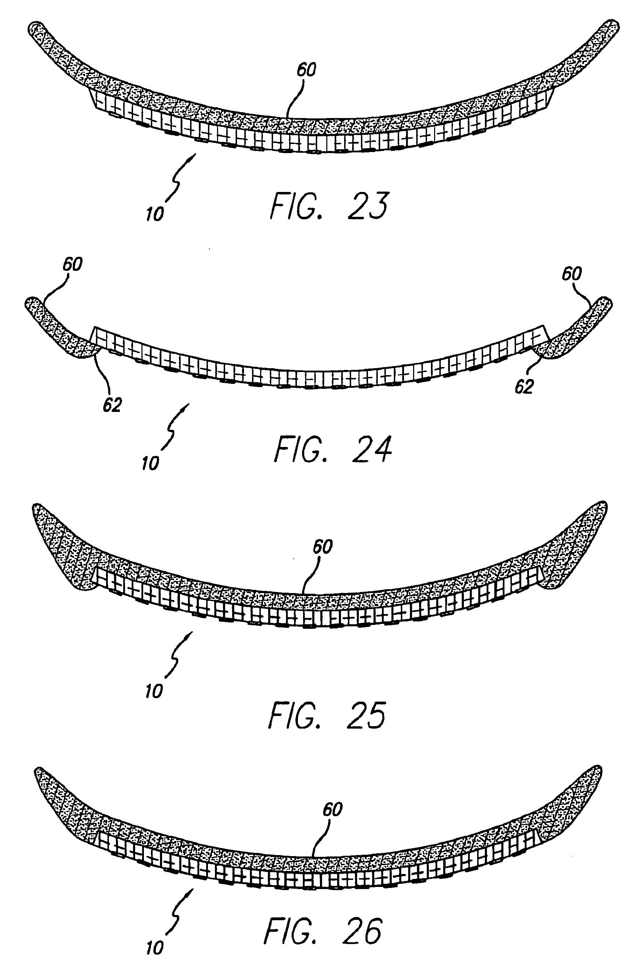

Fig. 23 depicts a flexible circuit array 10 with a protective skirt 60 bonded to the back side of the flexible circuit array 10 with a progressively decreasing radius and/or decreasing thickness toward the edges.

Fig. 24 depicts a flexible circuit array 10 with a protective skirt 60 bonded to the front side of the flexible circuit array 10 with a progressively decreasing radius and/or decreasing thickness toward the edges.

Fig. 25 depicts a flexible circuit array 10 with a protective skirt 60 bonded to the back side of the flexible circuit array 10 and molded around the edges of the flexible circuit array with a progressively decreasing radius and/or decreasing thickness toward the edges.

Fig. 26 depicts a flexible circuit array 10 with a protective skirt 60 bonded to the back side of the flexible circuit array 10 and molded around the edges of the flexible circuit array and flush with the front side of the array with a progressively decreasing radius and/or decreasing thickness toward the edges.

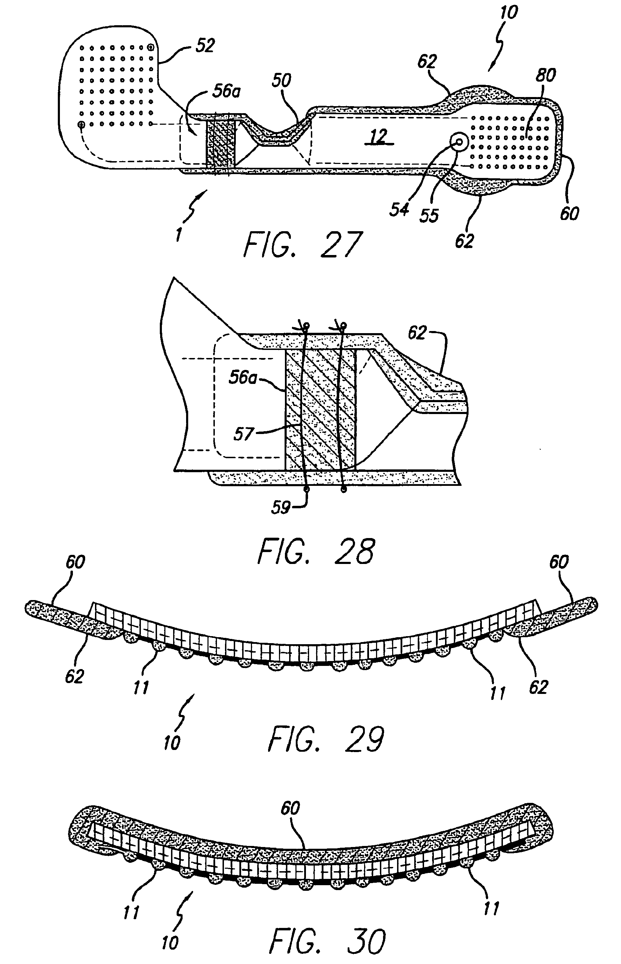

Fig. 27 depicts a side view of the array with a skirt 60 containing a grooved and rippled pad 56a instead a suture tab 56. This pad 56a has the advantage of capturing a mattress suture 57. A mattress suture 57 has the advantage of holding the groove or rippled pad 56a in two places as shown in Fig. 28. Each suture 57 is fixed on the tissue on two places 59. A mattress suture 57 on a grooved or rippled mattress 56a therefore provides a better stability.

Fig. 29 depicts a flexible circuit array 10 with a protective skirt 60 bonded to the front side of the flexible circuit array 10 with individual electrode 13 windows and with material, preferably silicone between the electrodes 13.

Fig. 30 depicts a flexible circuit array with a protective skirt bonded to the back side of the flexible circuit array and molded around the edges of the flexible circuit array with individual electrode windows and with material, preferably silicone between the electrodes 13.

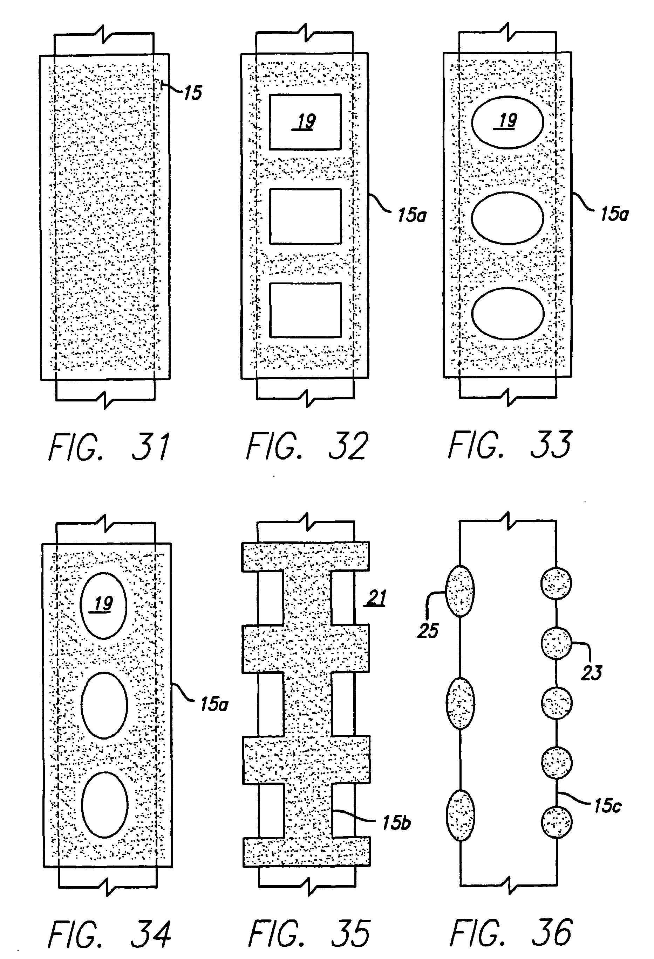

Figs. 31-36 show several surfaces to be applied to one or both sides of the flexible circuit array cable. The surfaces are thin films containing a soft polymer, preferably silicone. Fig. 31 shows a flange 15: A flange 15 can be a solid film of material containing silicone added to the surface of the polymer containing polyimide. Figs. 32-34 show a ladder 15a: A ladder 15a is a flange with material removed from central portions in some shape 19. Fig. 35 shows a skeleton structure 15b. A skeleton15b is a flange with material removed from perimeter portions in some shape 21. Fig. 36 shows a structure 15c with beads 23 and bumpers 25. A bead 23 is material added to perimeter portions of the polymer cable in some shape without material being added on the central area. A bumper 25 can be an extended or continuous version of the beaded approach. Both approaches are helpful in preventing any possible injury of the tissue by the polymer.

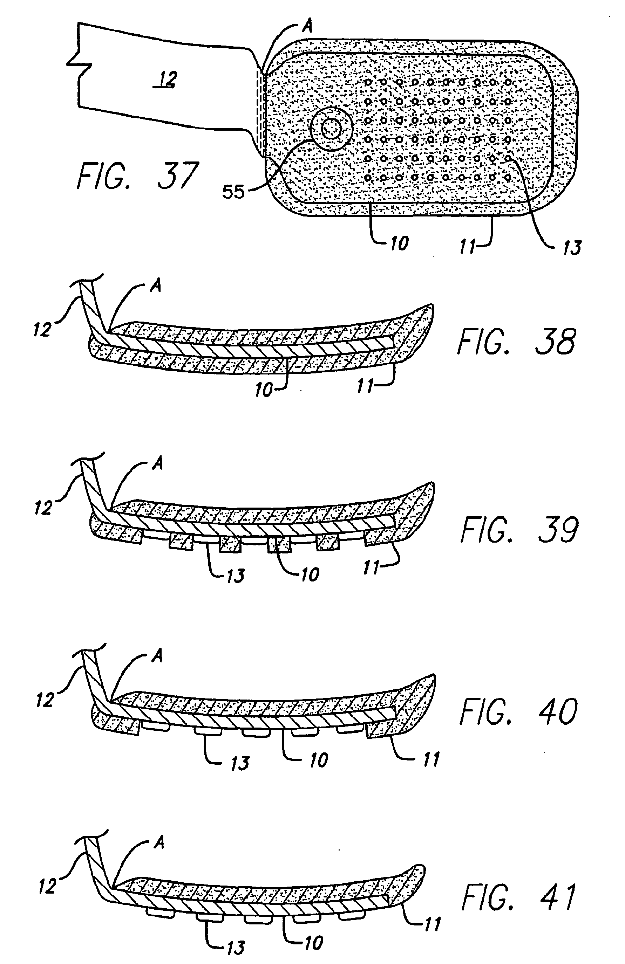

Fig. 37 depicts the top view of the flexible circuit array 10 being enveloped within an insulating material 11. The electrode array 10 comprises oval-shaped electrode array body 10, a plurality of electrodes 13 made of a conductive material, such as platinum or one of its alloys, but that can be made of any conductive biocompatible material such as iridium, iridium oxide or titanium nitride. The electrode array 10 is enveloped within an insulating material 11 that is preferably silicone. "Oval-shaped" electrode array body means that the body may approximate either a square or a rectangle shape, but where the corners are rounded. This shape of an electrode array is described in the U.S. Patent Application No. 20020111658, entitled "Implantable retinal electrode array configuration for minimal retinal damage and method of reducing retinal stress" and No. 20020188282, entitled "Implantable drug delivery device" to Robert J. Greenberg et al., the disclosures of both are incorporated herein by reference.

The material body 11 is made of a soft material that is compatible with the electrode array body 10. In a preferred embodiment the body 11 made of silicone having hardness of about 50 or less on the Shore A scale as measured with a durometer. In an alternate embodiment the hardness is about 25 or less on the Shore A scale as measured with a durometer.

Fig. 38 depicts a cross-sectional view of the flexible circuit array 10 being enveloped within an insulating material 11. It shows how the edges of the material body 11 are lifted off due to the contracted radius at the edges. The electrode array 10 preferably also contains a fold A between the cable 12 and the electrode array 10. The angle of the fold A secures a relief of the implanted material.

Fig. 39 depicts a cross-sectional view of the flexible circuit array 10 being enveloped within an insulating material 11 with open electrodes 13 and the material 11 between the electrodes 13.

Fig. 40 depicts a cross-sectional view of the flexible circuit array 10 being enveloped within an insulating material 11 with open electrodes 13. This is another embodiment wherein the electrodes 13 are not separated by the material 11 but the material 11 is extended.

Fig. 41 depicts a cross-sectional view of the flexible circuit array 10 being enveloped within an insulating material 11 with electrodes 13 on the surface of the material 11. This is a further embodiment with the electrode 13 on the surface of the material 11, preferably silicone. The embodiments shown in Figs. 39, 40, and 41 show a preferred body 11 containing silicone with the edges being lifted off from the retina due to contracted radius of the silicone body 11 at the edges.

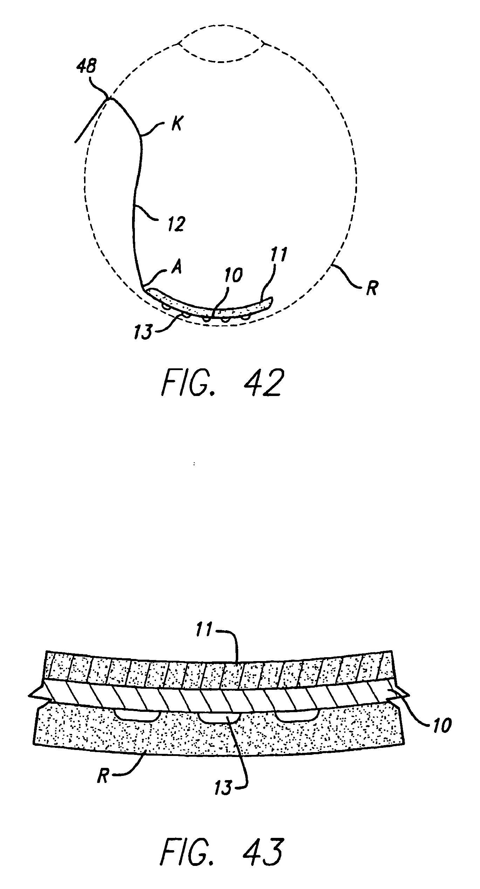

Fig. 42 depicts a cross-sectional view of the flexible circuit array 10 being enveloped within an insulating material 11 with electrodes 13 on the surface of the material 11 insight the eye with an angle K in the fold of the flexible circuit cable 12 and a fold A between the circuit electrode array 10 and the flexible circuit cable 12. The material 11 and electrode array body 10 are in intimate contact with retina R. The surface of electrode array body 10 in contact with retina R is a curved surface with a matched radius compared to the spherical curvature of retina R to minimize pressure concentrations therein. Further, the decreasing radius of spherical curvature of material 11 near its edge forms edge relief that causes the edges of the body 11 to lift off the surface of retina R eliminating pressure concentrations at the edges. The edge of body 11 is rounded to reduce pressure and cutting of retina R.

Fig. 43 shows a part of the Fig. 42 enlarged showing the electrode array 10 and the electrodes 13 enveloped by the polymer material, preferably silicone 11 in intimate contact with the retina R.

The electrode array 10 embedded in or enveloped by the polymer material, preferably silicone 11 can be preferably produced through the following steps. The soft polymer material which contains silicone is molded into the designed shape and partially hardened. The electrode array 10 which preferably contains polyimide is introduced and positioned in the partially hardened soft polymer containing silicone. Finally, the soft polymer 11 containing silicone is fully hardened in the designed shape enveloping the electrode array 10. The polymer body 11 has a shape with a decreasing radius at the edges so that the edges of the body 11 lift off from the retina R.

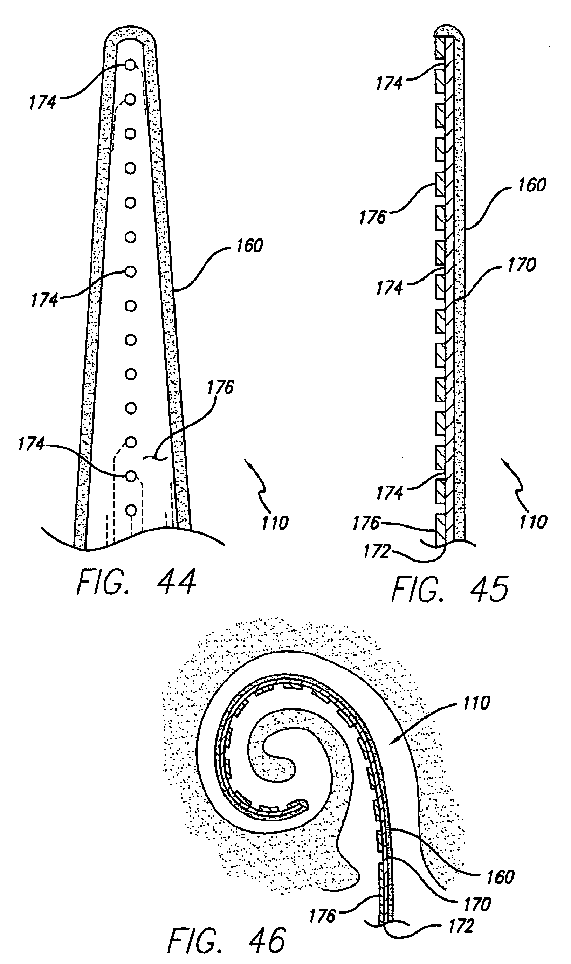

Figs. 44 - 46 show application of the present invention to a cochlear prosthesis. Fig. 44 shows of front view of cochlear electrode array 110. The cochlear electrode array 110 tapers toward the top to fit in an ever smaller cochlea and because less width is required toward the top for metal traces. The electrodes 174 are arranged linearly along the length of the array 110. Further a skirt 160 of more compliant polymer, such as silicone surrounds the array 110. Fig. 45 is a side view of the cochlear electrode array 110. The cochlear electrode array 110 includes a bottom polymer layer 170, metal traces 172 and a top polymer layer 176. Openings in the top polymer layer 176 define electrodes 174.

The cochlear electrode array 110 is made flat as shown in Figs. 44 and 45. It is then thermoformed, as described earlier, into a spiral shape to approximate the shape of the cochlea, as shown in Fig. 46. The cochlear electrode array 110 is implanted with the bottom layer 170 formed toward the outside of the curvature, and the top polymer layer 176 toward the inside of the curvature. This curvature is opposite of the curvature resulting from the thermoforming process used for a retinal array. A cortical array would be thermoformed to curve inward like a cochlear array.

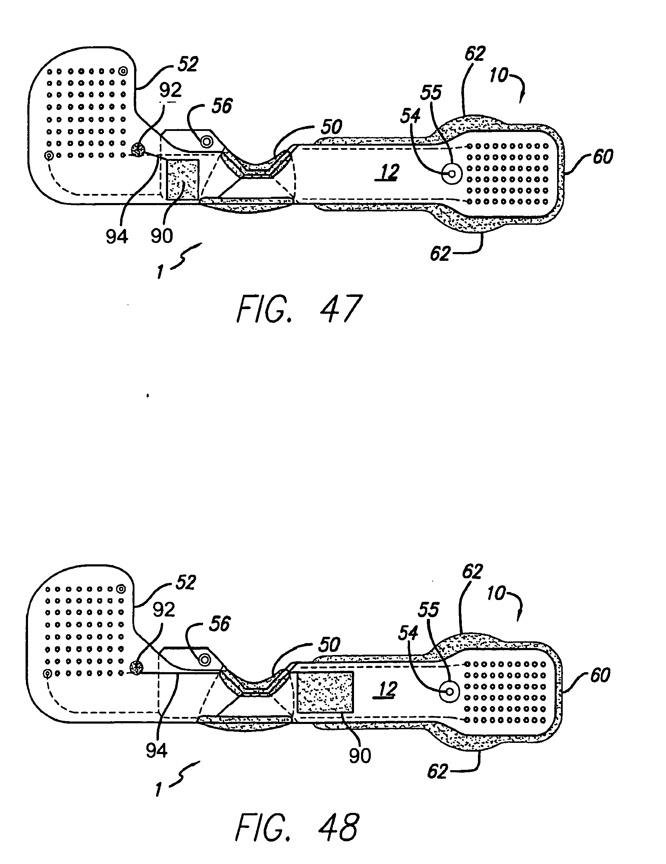

Fig. 47 shows the preferred electrode array with the return electrode 90 on the front of the cable outside the eye. The return electrode is coupled by a cable 94 to a contact pad 92 for attaching the return electrode to the electronics package.

Fig. 48 shows the preferred electrode array with the return electrode 90 on front of the cable inside the eye.

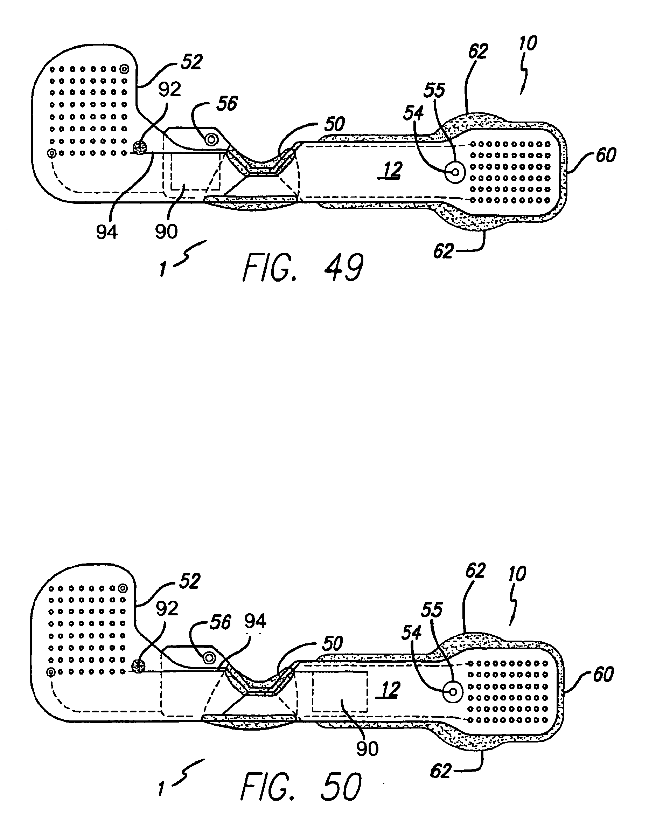

Figure 49 is the preferred electrode array with the return electrode 90 on the back of the cable outside the eye.

Fig. 50 is the preferred electrode array with the return electrode 90 on the back of the cable inside of the eye.

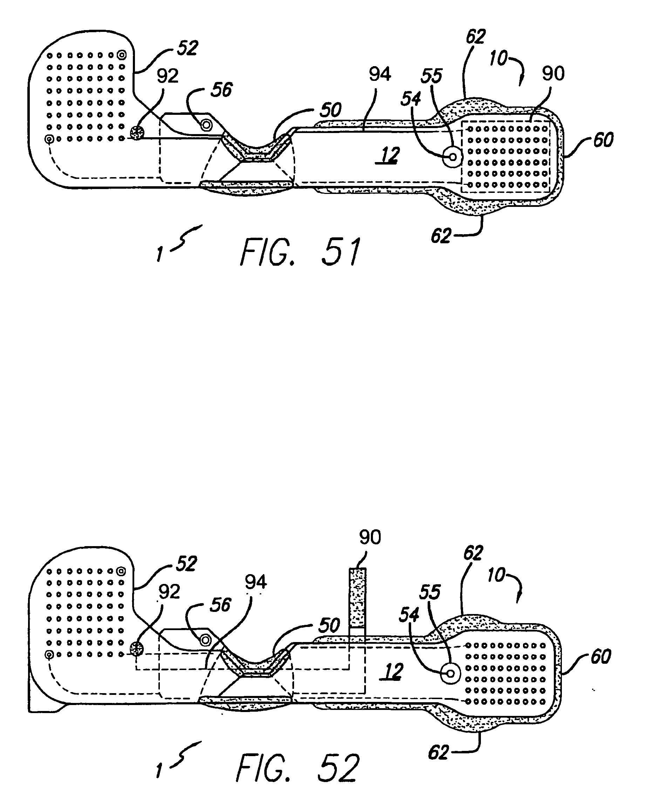

Fig. 51 is the preferred electrode array with the return electrode 90 on the back of the electrode array.

Fig. 52 is the preferred electrode array with the return electrode 90 on a separate cable inside the eye.

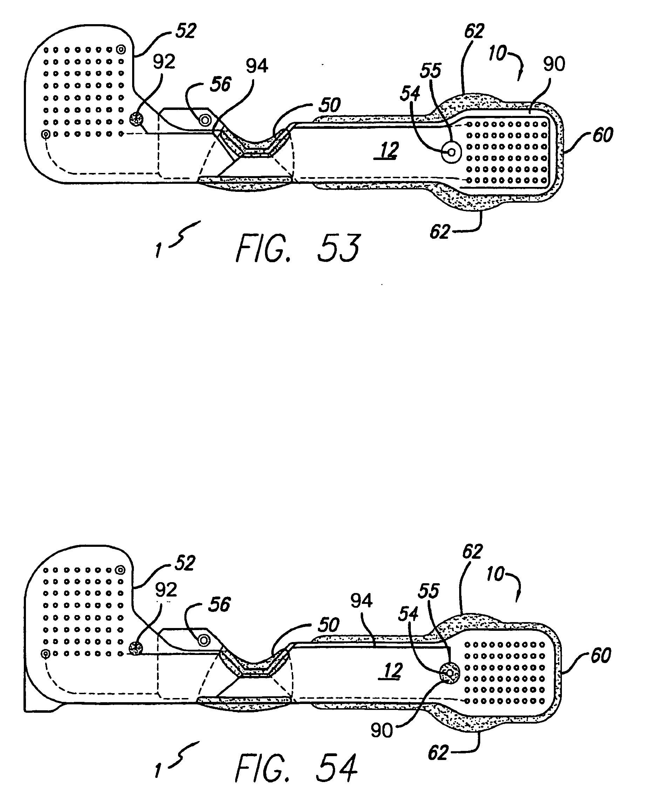

Fig. 53 is the preferred electrode array with the return electrode 90 on the face of the electrode array in a horseshoe pattern around the stimulating electrodes.

Fig. 54 is the preferred electrode array with the return electrode 90 around the tack hole and in electrical contact with the tack.

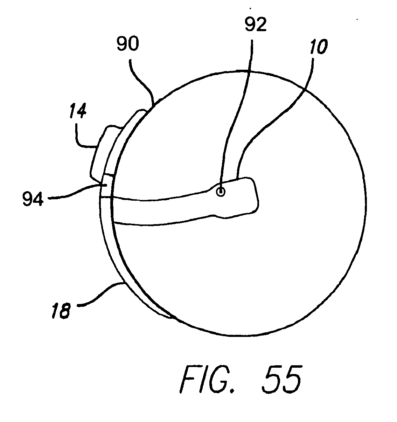

Fig. 55 is the preferred visual prosthesis with the return electrode 90 on the back of the secondary coil against the sclera. The return electrode 90 should be provided in a mesh, star pattern or hash pattern to reduce the eddy current effect on the coil.

It would also be advantageous to provide more than one of the return electrodes described herein and provide a switch mechanism to switch between or utilize more than one. This way a user could select the configuration that is most comfortable for them.

This invention addresses this problem by using a multi-electrode array as multi-return electrodes placed outside the eyeball and directly under the active epi-retina array. It will greatly reduce the sensitivity of the electrode-tissue distance to stimulation results. The requirement of firm contact of electrodes to the retina is relieves. Therefore the risk of damaging the delicate retina by placing the electrode array on its surface is reduced. The return electrode can be implanted routinely during the surgery.

This invention involves an electrode array used as multi-return electrodes. The method involves controlling the stimulating current flow between the active electrodes and the return electrodes, and alternative ways to implement the multi-return electrode array. The muti-return electrode comprises an array of electrode discs with larger sizes than the discs of the active electrodes. The electrode discs can be made of safe electrode materials similar to or same as that of the active electrodes. The number of return electrode discs in the array can be a fraction of the active electrodes and may vary depending on the manufacturing flexibility.

Similarly, the discs are only exposed in the array surface facing the eyeball. The back side of the array is sealed. The current flowing to the return electrodes can only come from one direction. The return array needs to be in firm contact with the outside surface of the eyeball. The firm contact can be achieved by suturing. In order to achieve the best result, the return array needs to be placed directly underneath the active array such that the overlapping area between the two arrays is at its largest.

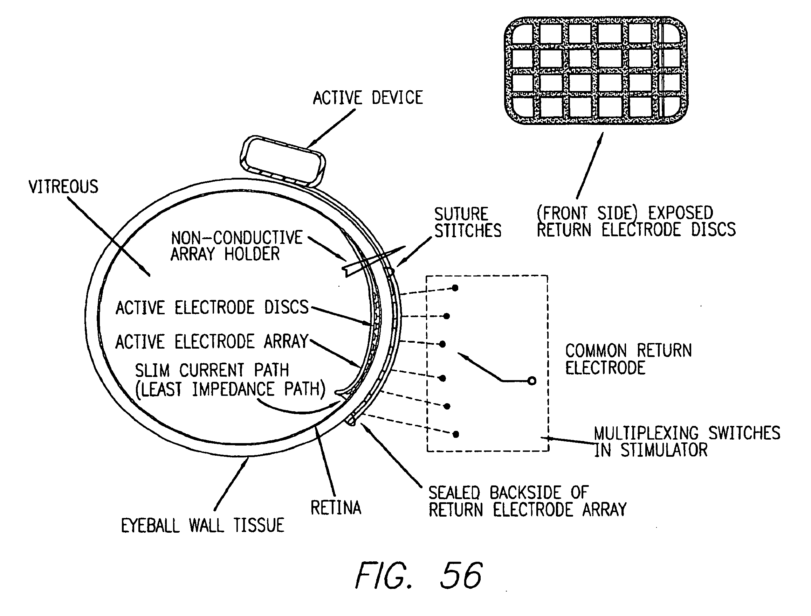

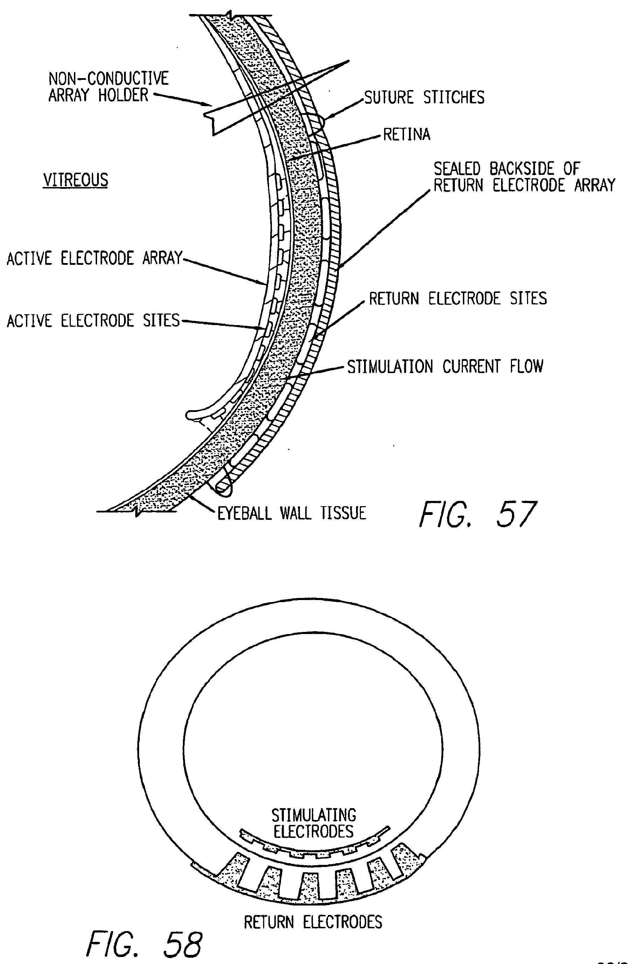

However, perfect alignment of the arrays is not necessary, because any small misalignment will be corrected automatically by the subject's perception adaptation. Electrically, each return electrode is connected to the common return electrode through a multiplexing switch which is turned off when the electrode is inactive. During each stimulation pulse, a small group of active electrodes defined by the prosthesis controller will pair with the closest return electrode in the return array to conduct stimulation as shown in Figures 56 and 57.

The pairing return electrode is connected to the current return path in the implant stimulator, while other return electrodes are turned off from it. Thus the currents from the stimulating electrodes will pass through the retina and reach the paired return electrode through the shortest and also the lowest impedance path. For other stimulation pulses other groups of active electrodes are paired with their corresponding closet return electrodes, and so on.

If an active electrode is slightly lifted from the retina surface, the current will still track the similar path and reach the return electrode as long as the size of the return electrode is comparable to the active. On the other hand, active electrodes can choose any return electrode to pair with, making it possible to create virtual electrode mapping. Alternatively, the return electrodes may also be connected to active stimulation drivers of the implant stimulator to conduct through-retina bipolar stimulation. Physically, the multi-return array can be implemented with thin film technologies, thin wire electrode techniques or directly be plated or sticked to the inner surface of the implant coil overmold if a coil is used this way.

Multi Return Electrode for Visual Prosthesis shows the following advantages.
a) Great reduction or elimination of the sensitivity of the electrode-tissue distance to stimulation results and assure individual electrode percepts.
b) Improved spatial resolution comparing a single return electrode.
c) In achieving steps a) and b) a firm contact of electrodes to the retina is not required and risk of damaging of the retina by placing the electrode array on its surface is reduced.
d) Virtual electrode mapping for the epi-retina stimulator is possible.
e) Through-retina bipolar stimulation is possible.
f) Surgery requirement can be met more easily comparing to sub-retina implementation.

Fig. 56 depicts a cross sectional vie of epi-retina prosthesis configuration using multi-return electrode array placed on the eye wall outside the eye. It shows an epi-retinal prosthesis configuration using multi return electrode array placed on the eye wall outside of the eye. Inside the eye ball (the vitreous) are placed non-conductive array holder, active electrode discs, active electrode array, and the stimulating current path. Outside of the eye ball the sealed backside of the return electrode (common return electrode) array is placed. Further, the front side exposed return electrode discs are shown.

Fig. 57 depicts an enlarged portion of the configuration shown in Figure 56.

Fig. 58 depicts a cross sectional view of an eye with stimulating and return electrodes. An example of penetrating electrode array with rod electrodes is shown.

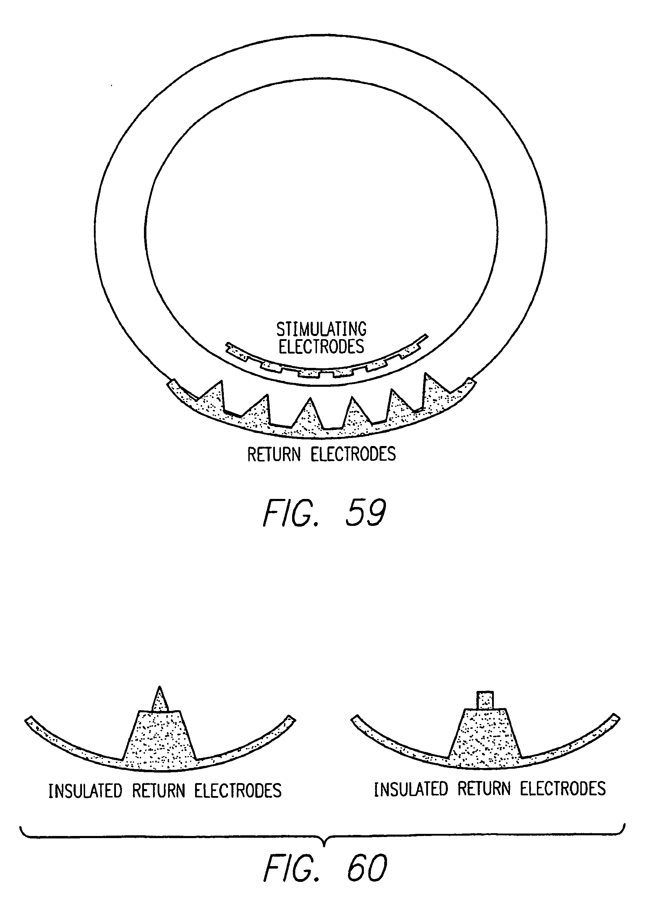

Fig. 59 depicts a cross sectional view of an eye with stimulating and return electrodes. An example of penetrating electrode array with pointed electrodes is shown.

Fig. 60 depicts an elevated cross-sectional view of insulated return electrodes. Examples of penetrating electrode array with coated rod and pointed electrodes are shown.

Return electrodes need to be sealed in the backside and the array needs to be sutured to the outside eye wall to guarantee the desired least impedance current paths from the active electrodes to the return electrode discs. The return and active arrays need not be aligned perfectly. A misalignment can be easily corrected using calibration. Tack or other means for fixing of the active array shall not contain conductive material so that the stimulation current paths are not distracted.

The ability to cause a percept by stimulation is not affected by a "noncontact" electrode or the distance of the active array to the retina surface. The effective pixel resolution depends on the size of the return electrode discs. The smaller the size of the return electrode is the better is the result. The effects of active electrode array movement on percepts identification also depend on the sizes of the return electrode discs. Smaller discs should have lower effects. The sizes of the return electrodes will affect the electrode impedance, but in a less significant way as the sizes of the active electrodes.

The stimulation shall be rastered between the groups of stimulation against different return electrodes. Synchronous stimulation onto different return electrodes is also possible with special circuits. Virtual electrode realization is possible using the multi-return electrode array method, which is potentially important for resolution enhancement and real word visual recognition.

The return electrode array can also be placed on the inner surface of the implant coil receiving the RF power through the inductive link. The electrical implementation of the multi-return in the implanted stimulator can contain common multiplexed switches. The relaxed requirement of active electrode array contacting the retina surface reduces the risk of retina damage by the electrode array.

In the proposed design, the return electrodes are positioned to create lowest impedance paths to guide current flow through retinal cells. The return electrodes will penetrate the eye wall tissue from the back of the eye and will only penetrate through the sclera tissue but not the retina. The back of the return electrode array is insulated. The penetrating tips will also help to stabilize the return electrode in position. A remote big return electrode may still be necessary for shorting purposes.

Examples of penetrating the return electrodes are shown in Figs. 58 and 59. Other shapes not shown in the Figures can also be used. The pointed electrodes can be coated with an insulator to have only the tips exposed as shown in Fig. 60, to further reduce current leakage to the sides.

Performance of electrical stimulation of neurons may depend on the electric field distribution from each electrode. It is believed that a more focused electric field with improved performance reduces threshold charge. In this sense a focal and return electrode or electrode array may be highly advantageous.

For retinal neuron stimulators of the epiretinal configuration, a focal return electrode or array would be placed posterior of the sclera aligned with the epiretinal stimulation array. Alignment could be achieved using concentric fixation structures, such as a tack, or the return electrode could be contained within a somewhat rigid housing connected to the other extra ocular implant components, like band or coil.

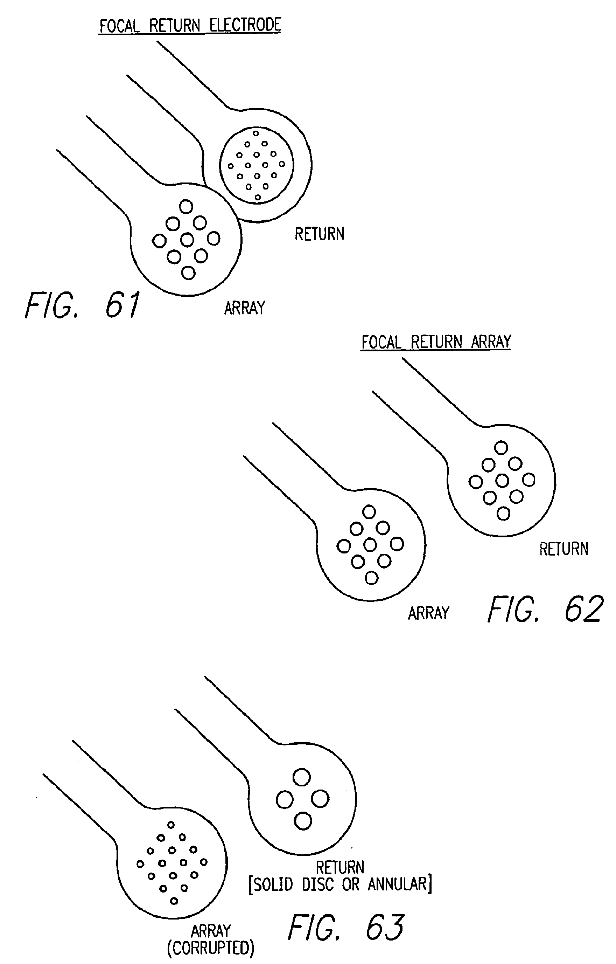

Fig. 61 depicts a top view on a focal return electrode having a similar perimeter as the electrode array. It is shown the comparison between the electrode array and the return electrode.

Figs. 62 and 63 show the match or small group of electrodes. In Fig. 62 a focal return electrode matches individual electrodes. In Fig. 63 depicts a focal return electrode matches small groups of electrodes.

The invention further involves an implantable electronic system wherein the significant mechanical and insulative functions are achieved with a single family of polymer materials. Those materials are thermoplastic and are available in thin sheets, possess low moisture uptake, are suitable for implantation and for multilayer construction with strong chemical bonding between the layers, for example liquid crystal polymer (LCP). Further examples of promising materials include polybenoxazole, polynorbornene, polyethylene naphthalate, fluorinated polymers and polyimides.

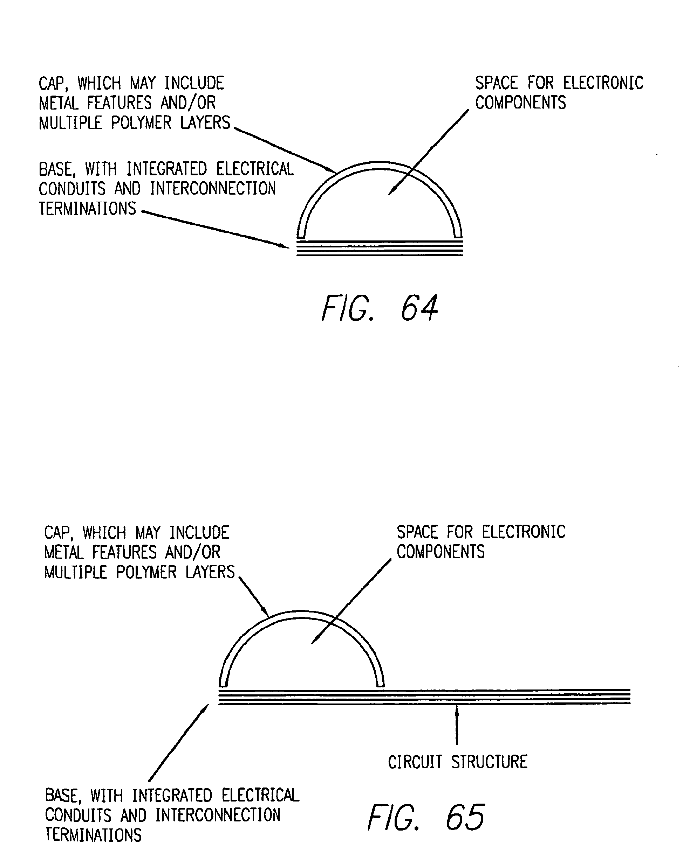

Fig. 64 depicts a cross sectional view of a packaging for an implantable device, which consists of a discrete package. The base of the package is a single or multilayer structure with integrated electrical conduits terminating at both front side and back side connecting points. The enclosure is created by a dome or cap shaped single or multilayer structure, with or without integrated electrical features, bonded on the case.

FIG. 65 depicts a cross sectional view of a packaging for an implantable device, which consists of a flexible circuit like structure. This circuit is a single or multilayer structure with integrated electrical conduits terminating at either on the front side and back side connecting points both in the area of the "package" and elsewhere along its body, for instance at electrode sites. The enclosure is created in one area of the circuit by a dome or cap shaped as a single or multilayer structure, with or without integrated electrical features, bonded to the circuit.

Potential applications for these structures include passive and/or active sensor and/or actuator devices, like implantable neural stimulators, recording devices, drug deliver apparatuses and other prosthetics.

A discrete embodiment results in a package architecture that is freestanding and ready for assembly with additional components. An integrated embodiment results in a package architecture that already incorporates additional device components.

An alternative is an all gold metal conductor scheme. Sufficient insulation by the polymer encapsulation prevents exposure of the gold conductors to any corrosive environment. Electron migration is prevented so long as the polymer interfaces are strongly bonded. The advantage of gold in comparison to platinum or iridium is the simplified processing and increased design flexibility gained by gold. The gold conductors at exposed electrode sites, where charge transfer is desired, are over coated or otherwise protected by superior electrochemical materials.

Accordingly, what has been shown is an improved method making a neural electrode array and improved method of stimulating neural tissue. While the invention has been described by means of specific embodiments and applications thereof, it is understood that numerous modifications and variations could be made thereto by those skilled in the art without departing from the scope of the invention. It is therefore to be understood that within the scope of the claims, the invention may be practiced otherwise than as specifically described herein.

## Claims

1. A flexible circuit electrode array (10) comprising:
a polymer base layer (70);
metal traces (72) deposited on said polymer base layer, including stimulating electrodes (74) suitable to stimulate retinal neural tissue;
a polymer top layer (76) deposited on said polymer base layer and said metal traces;
a return electrode;
a first fixation structure (54) suitable to fix the flexible circuit electrode array to an epiretinal surface; and
**characterized in that** the return electrode (90) is separated from said stimulating electrodes and includes a second fixation structure suitable to fix the return electrode to the outside of a sclera and is coupled by a cable (94) to a contact pad (92) for attaching the return electrode to an electronics package (14).

2. The flexible circuit electrode array according to claim 1, wherein a large return electrode is suitable to be fixed outside of the sclera and directly under the stimulating electrodes.

3. The flexible circuit electrode array according to any of claims 1 or 2, wherein alignment between the return electrode and the stimulation electrodes is achieved by concentric fixation structures.

4. The flexible circuit electrode array according any of claims 1 to 3, wherein the return electrode is contained within a rigid housing connected to an extra ocular implant components.

5. The flexible circuit electrode array according to claim 4, wherein the rigid housing includes band and/or coil.

6. The flexible circuit electrode array according to any of claims 1 to 5, wherein the return electrode is configured to be placed on the front of a cable outside of the eye.

7. The flexible circuit electrode array according to any of claims 1 to 6, wherein the return electrode is configured to be sutured to the outside of the sclera.

8. The flexible circuit electrode array according to any of claims 1 to 7, wherein the return electrode is configured to be placed on the back of a secondary coil against the sclera.

## Patentansprüche

1. Flexible Schaltungselektrodenanordnung (10), die Folgendes umfasst:
eine Polymerbasisschicht (70),
Metallbahnen (72), die auf der Polymerbasisschicht abgeschieden sind, einschließlich stimulierender Elektroden (74), die zur Stimulation von Nervengewebe der Netzhaut geeignet sind;
eine Polymerdeckschicht (76), die oberhalb der Polymerbasisschicht und der Metallbahnen abgeschieden ist;
eine Neutralelektrode;
eine erste Befestigungsstruktur (54), die geeignet ist, um die flexible Schaltungselektrodenanordnung an einer epiretinalen Oberfläche zu befestigen, und
**dadurch gekennzeichnet, dass** die Neutralelektrode (90) von den stimulierenden Elektroden getrennt ist und eine zweite Befestigungsstruktur umfasst, die geeignet ist, um die Neutralelektrode außen an einer Sklera zu befestigen, und die mit einem Kabel (94) mit einem Kontaktfeld (92) verbunden ist, um die Neutralelektrode mit einer Elektronikbaugruppe (14) zu verbinden.

2. Flexible Schaltungselektrodenanordnung nach Anspruch 1, worin eine große Neutralelektrode geeignet ist, um außen an der Sklera und direkt unterhalb der stimulierenden Elektroden angebracht zu werden.

3. Flexible Schaltungselektrodenanordnung nach Anspruch 1 oder 2, worin die Ausrichtung von Neutralelektrode und stimulierenden Elektroden durch konzentrische Befestigungsstrukturen erfolgt.

4. Flexible Schaltungselektrodenanordnung nach einem der Ansprüche 1 bis 3, worin die Neutralelektrode in einem starren Gehäuse aufgenommen ist, das mit zusätzlichen Augenimplantatkomponenten verbunden ist.

5. Flexible Schaltungselektrodenanordnung nach Anspruch 4, worin das starre Gehäuse ein Band und/oder eine Spule umfasst.

6. Flexible Schaltungselektrodenanordnung nach einem der Ansprüche 1 bis 5, worin die Neutralelektrode ausgebildet ist, um am Ende eines Kabels außen am Auge angeordnet zu werden.

7. Flexible Schaltungselektrodenanordnung nach einem der Ansprüche 1 bis 6, worin die Neutralelektrode ausgebildet ist, um durch eine Naht außen an der Sklera angebracht zu werden.

8. Flexible Schaltungselektrodenanordnung nach einem der Ansprüche 1 bis 7, worin die Neutralelektrode ausgebildet ist, um hinten an einer Sekundärspule in Richtung der Sklera ausgerichtet angeordnet zu werden.

## Revendications

1. Groupement d'électrodes de circuit flexible (10) comprenant:
une couche de base en polymère (70);
des traces métalliques (72) déposées sur ladite couche de base en polymère, incluant des électrodes de stimulation (74) aptes à stimuler le tissu rétinal neural;
une couche supérieure en polymère (76) déposée sur ladite couche de base en polymère et lesdites traces métalliques;
une électrode de retour;
une première structure de fixation (54) apte à fixer le groupe d'électrodes de circuit flexible à une surface épirétinale; et
**caractérisé en ce que** l'électrode de retour (90) est séparée desdites électrodes de stimulation et comprend une deuxième structure de fixation apte à fixer l'électrode de retour à l'extérieur d'une sclère et est couplée par un câble (94) à un coussinet de contact (92) pour fixer l'électrode de retour à un boîtier électronique (14).

2. Groupement d'électrodes de circuit flexible selon la revendication 1, où une grande électrode de retour est apte à être fixée à l'extérieur de la sclère et directement sous les électrodes de stimulation.

3. Groupement d'électrodes de circuit flexible selon l'une quelconque des revendications 1 ou 2, où l'alignement entre l'électrode de retour et les électrodes de stimulation est atteint par des structures de fixation concentriques.

4. Groupement d'électrodes de circuit flexible selon l'une quelconque des revendications 1 à 3, où l'électrode de retour se trouve dans un boîtier rigide connecté à des composants d'implants oculaires supplémentaires.

5. Groupement d'électrodes de circuit flexible selon la revendication 4, où le boîtier rigide comprend une bande et/ou une bobine.

6. Groupement d'électrodes de circuit flexible selon l'une quelconque des revendications 1 à 5, où l'électrode de retour est configurée pour être placée sur l'avant d'un câble à l'extérieur de l'oeil.

7. Groupement d'électrodes de circuit flexible selon l'une des revendications 1 à 6, où l'électrode de retour est configurée pour être suturée à l'extérieur de la sclère.

8. Groupement d'électrodes de circuit flexible selon l'une quelconque des revendications 1 à 7, où l'électrode de retour est configurée pour être placée sur l'arrière d'une bobine secondaire contre la sclère.
